# EUROPEAN PATENT APPLICATION

(11) **EP 4 620 404 A1**
(43) Date of publication of application: **24.09.2025**
(21) Application number: 25164757.4
(22) Date of filing: 19.03.2025
(51) Int. Cl.: A61B 8/08, A61B 8/00

(54) **METHOD FOR PROVIDING USER INTERFACE FOR CARDIAC ULTRASOUND IMAGING GUIDELINE AND DEVICE FOR PROVIDING USER INTERFACE USING THE SAME**

(30) Priority: 20.03.2024 KR 20240038637; 14.03.2025 KR 20250033404
(71) Applicant: Ontact Health Co., Ltd., Seoul 03764 (KR)
(72) Inventor: LEE, Seung Ah, Seoul (KR); JEON, Jae Ik, Seoul (KR); JEONG, Hyun Seok, Seoul (KR)
(74) Representative: Müller Schupfner & Partner Patent- und Rechtsanwaltspartnerschaft mbB (Muc)

(57) **Abstract**

The present disclosure provides a method for providing a user interface for a cardiac ultrasound image guideline implemented by a processor, the method including receiving a cardiac ultrasound image of a subject, determining probe guidance according to movement of an ultrasound probe from the cardiac ultrasound image, and providing a user interface regarding the probe guidance according to the movement of the ultrasound probe, and a device using the same.

## Description

### CROSS-REFERENCE TO RELATED APPLICATIONS

This application claims the priority of Korean Patent Applications No. 10-2025-0033404 filed on March 14, 2025 and No. 10-2024-0038637 filed on March 20, 2024, in the Korean Intellectual Property Office, the disclosure of which is incorporated herein by reference.

### BACKGROUND

### Field

The present disclosure relates to a method for providing a user interface for a cardiac ultrasound image guideline and a device for providing a user interface for a cardiac ultrasound image guideline using the same.

### Description of the Related Art

A cardiac ultrasound examination is performed by projecting ultrasound waves on a three-dimensional structure of a heart in multiple planes to obtain images of the heart and measure hemodynamic variables.

In this case, a medical staff positions an ultrasound probe in a location where it is easy to obtain ultrasound images, so as to obtain multi-faceted images through anatomical structures around the heart, such as between ribs, and records the images by finding the appropriate slice through rotation and tilting.

Furthermore, a measurement value associated with morphological changes in the heart can be determined from appropriate cross-sectional images, and specific diseases can be diagnosed from the measurement value. In other words, the measurement value can be provided as clinical values for diagnosing heart diseases and further for observing prognosis.

Meanwhile, the determination of the measurement value through the cardiac ultrasound examination is dependent on the interpretation of the medical staff, and the reliability of the results may also depend on the skills of the medical staff.

That is, the accuracy of the cardiac ultrasound examination may vary greatly depending on the skill level of the medical staff, so there is a continuous demand for the development of a new user interface providing system that can obtain highly reliable cardiac ultrasound images.

The background technology of the disclosure has been written to facilitate understanding of the present disclosure. It should not be understood that the matters described in the background technology of the disclosure are recognized as prior art.

### SUMMARY

Meanwhile, in order to solve the above-mentioned problem, the inventors of the present disclosure have developed a user interface providing system that displays various information on cardiac ultrasound imaging guidelines for cardiac ultrasound images.

The inventors of the present disclosure have recognized that by implementing the new user interface providing system, it is possible to solve the problem of conventional processes having difficulty in determining a measurement value due to time constraints in emergency situations.

In particular, the inventors of the present disclosure have recognized that by applying an artificial neural network, a clinical process can be supplemented to provide information by selecting a frame in which the measurement value can be determined without additional work for mode switching, and highly reliable information can be provided.

Accordingly, the inventors of the present disclosure have expected that by providing the new user interface providing system, it is possible to provide highly reliable analysis results for cardiac ultrasound images regardless of the skill level of the medical staff.

In particular, the inventors of the present disclosure have expected that when the new user interface providing system is applied to a small device including a handheld type that has limitations in obtaining highly reliable cardiac ultrasound images, fast and highly accurate decision making is possible.

Furthermore, the inventors of the present disclosure have provided information on cross-sectional views for cardiac ultrasound images and further provided information on operation of an ultrasound device for probe guidance required for the cross-sectional views.

Accordingly, a subject of the present disclosure is to provide a user interface providing method configured to provide a user interface that displays information on probe guidance from a received cardiac ultrasound image, and a device using the same.

Subjects of the present disclosure are not limited to the subjects mentioned above, and other subjects not mentioned will be clearly understood by those skilled in the art from the description below.

In order to achieve the aforementioned subjects, a method for providing a user interface for a cardiac ultrasound image guideline according to one embodiment of the present disclosure is provided.

The method is a method for providing a user interface for a cardiac ultrasound image guideline implemented by a processor, including: receiving a cardiac ultrasound image of a captured subject; determining probe guidance according to movement of an ultrasound probe based on the cardiac ultrasound image; and providing a user interface including the probe guidance.

According to an aspect of the present disclosure, the determining the probe guidance may include determining first probe guidance or second probe guidance according to movement, and the user interface may include an area where the first probe guidance or the second probe guidance is displayed.

According to another aspect of the present disclosure, the first probe guidance may include at least one probe operation guidance of standard, Probe Head Up, Probe Head Down, Probe Head left, Probe Head Right, Probe Rotate Clockwise, and Probe Rotate Counter-clockwise, and the second probe guidance may include at least one probe operation guidance of standard, Rib Up, Rib Down, Slide Left, and Slide Right.

According to still another aspect of the present disclosure, the second probe guidance may be defined as requiring larger probe movements than the first probe guidance, and the determining the probe guidance may include determining suitability for the first probe guidance, and determining the second probe guidance when the suitability for the first probe guidance is equal to or more than a predetermined level The providing the user interface may include providing a user interface including the second probe guidance.

According to still another aspect of the present disclosure, the determining probe guidance may further include determining a cross-sectional view for the cardiac ultrasound image, and determining probe guidance corresponding to the cross-sectional view, and the user interface may include an area where the cross-sectional view is displayed, and an area where the probe guidance corresponding to the cross-sectional view is displayed.

According to still another aspect of the present disclosure, the user interface may further include an area for displaying suitability according to the provision of the probe guidance corresponding to the cross-sectional view.

According to still another aspect of the present disclosure, the user interface may further include an area for displaying a hold state or an area for activating an auto recording state, and the providing the user interface may include providing the area for displaying the hold state or the area for activating the auto recording state when the movement of the probe corresponds to the probe guidance.

According to still another aspect of the present disclosure, the user interface may further include an area in which the received cardiac ultrasound image is displayed.

According to still another aspect of the present disclosure, the method may further include, after the providing the user interface, providing a blinking effect for an area where the cardiac ultrasound image is displayed when a probe is operated according to the probe guidance to obtain an optimal cardiac ultrasound image.

According to still another aspect of the present disclosure, the method may further include determining each of anatomical structures of the heart in the received cardiac ultrasound image and performing an evaluation on each of the anatomical structures, in which the user interface may include an area where an evaluation result is displayed.

According to still another aspect of the present disclosure, the performing the evaluation may include determining a structural score defined as a score for evaluating capture of an anatomical structure for the cardiac ultrasound image received based on a segmentation result, and determining an image quality score defined as a score for evaluating an image quality of the cardiac ultrasound image, in which the area where the evaluation result is displayed may include a structural score display area and a quality score display area.

According to still another aspect of the present disclosure, the structural score display area may be configured to visually display the structural score at a location corresponding to the anatomical structure based on the segmentation result, and the quality score display area may be configured to display the quality score in a bar shape.

According to still another aspect of the present disclosure, the user interface may further include an area for displaying a stored cardiac ultrasound image capable of storing and managing the captured cardiac ultrasound image, and the stored cardiac ultrasound image may include information on a captured time and cross-sectional view.

According to still another aspect of the present disclosure, the determining the probe guidance may include determining the ultrasound probe guidance based on the received cardiac ultrasound image by using a prediction model trained to output ultrasound probe guidance according to the movement of the ultrasound probe by inputting the cardiac ultrasound image.

In order to solve the aforementioned subjects, a device for providing a user interface for a cardiac ultrasound image guideline according to another embodiment of the present disclosure is provided.

The device includes: a communication unit configured to receive a cardiac ultrasound image of a captured subject; and a processor and a display unit functionally connected to the communication unit, in which the processor may be configured to determine probe guidance according to movement of an ultrasound probe based on the cardiac ultrasound image, and the display unit may be configured to display a user interface including the probe guidance.

According to an aspect of the present disclosure, the processor may be further configured to determine first probe guidance or second probe guidance according to movement, and the user interface may include an area where the first probe guidance or the second probe guidance is displayed.

According to another aspect of the present disclosure, the processor may be configured to determine suitability for the first probe guidance and determine the second probe guidance when the suitability for the first probe guidance is equal to or higher than a predetermined level, and the display unit may be further configured to display a user interface including the second probe guidance.

According to still another aspect of the present disclosure, the processor may be further configured to determine a cross-sectional view for the cardiac ultrasound image and determine probe guidance corresponding to the cross-sectional view.

According to still another aspect of the present disclosure, the processor may be further configured to provide the area for displaying the hold state or the area for activating the auto recording state when movement of a probe corresponds to the probe guidance.

According to still another aspect of the present disclosure, the processor may be further configured to determine each of the anatomical structures of the heart in the received cardiac ultrasound image, and perform an evaluation on each of the anatomical structures.

Specific details of other embodiments are included in the detailed description and drawings.

The present disclosure provides the user interface for the cardiac ultrasound image, thereby enabling the user to receive real-time guidance on optimal ultrasound probe movement. This enables the user to increase the accuracy of probe operation and obtain more reliable cardiac ultrasound images.

The present disclosure can provide appropriate probe guidance according to the ultrasound probe movement, and can improve user experience by additionally providing more precise guidance when a specific probe operation is suitable to be equal to or more than a predetermined level.

In addition, the present disclosure can support a user to perform an appropriate operation in real time by clearly providing probe guidance corresponding to a cross-section view on the user interface.

The present disclosure provides a visual indication of the suitability of probe guidance according to a cross-sectional view, thereby enabling a user to immediately recognize the current operating state and quickly perform necessary adjustments.

Furthermore, the present disclosure can increase the convenience of image acquisition by including the function of activating a hold state or an auto recording state, thereby enabling recording to be performed immediately when the user completes an appropriate probe operation.

The present disclosure can visually provide a structural score and an image quality score by evaluating the capture and image quality of anatomical structures in the cardiac ultrasound image. Through this, a user can immediately evaluate the quality of a captured image and perform additional operation if necessary.

In addition, the present disclosure is configured to manage the stored cardiac ultrasound image, thereby enabling efficient management of image data by supporting the retrieval of past images together with information on the shooting time and cross-sectional view.

The effects according to the present disclosure are not limited to those exemplified above, and more various effects are included in this specification.

The effects of the present disclosure are not limited to the aforementioned effects, and other effects, which are not mentioned above, will be apparently understood to a person having ordinary skill in the art from the following description.

The subjects to be achieved by the present disclosure, the means for achieving the subjects, and the effects of the present disclosure described above do not specify essential features of the claims, and, thus, the scope of the claims is not limited to the disclosure of the present disclosure.

### BRIEF DESCRIPTION OF DRAWINGS

The above and other aspects, features and other advantages of the present disclosure will be more clearly understood from the following detailed description taken in conjunction with the accompanying drawings, in which:
FIGS. 1A and 1B illustrate a system for providing a user interface for a cardiac ultrasound image guideline using a device for providing a user interface for a cardiac ultrasound image guideline according to various embodiments of the present disclosure.
FIG. 2A is a block diagram illustrating the configuration of a user device according to one embodiment of the present disclosure.
FIG. 2B is a block diagram illustrating the configuration of a server for providing a user interface according to one embodiment of the present disclosure.
FIGS. 3 and 4 illustrate a procedure of a method for providing the user interface for the cardiac ultrasound image guideline according to various embodiments of the present disclosure.
FIGS. 5 to 7, FIGS. 8A and 8B, FIGS. 9A to 9C, and 10 exemplarily illustrate a user interface according to methods for providing the user interfaces in various embodiments of the present disclosure.
FIG. 11 illustrates a procedure of a method for providing a user interface for a cardiac ultrasound image guideline based on a prediction model according to various embodiments of the present disclosure.
FIGS. 12A to 12L exemplarily illustrate a method for providing a user interface and the user interface according to more various embodiments of the present disclosure.
FIG. 13 illustrates a procedure of a method for providing a user interface for a cardiac ultrasound image guideline based on a prediction model according to more various embodiments of the present disclosure.

### DETAILED DESCRIPTION OF THE EMBODIMENT

Hereinafter, the exemplary embodiment of the present disclosure will be described with reference to the accompanying drawings and exemplary embodiments as follows. Scales of components illustrated in the accompanying drawings are different from the real scales for the purpose of description, so that the scales are not limited to those illustrated in the drawings.

The advantages of the disclosure and the method for achieving them will become apparent by referring to the embodiments described in detail below together with the accompanying drawings. However, the present disclosure is not limited to the embodiments disclosed below, but may be implemented in various different forms, and these embodiments are provided only to make the disclosure of the present disclosure complete and to fully inform those skilled in the art of the scope of the disclosure.

The shapes, sizes, ratios, angles, numbers, or the like disclosed in the drawings for explaining embodiments of the present disclosure are exemplary, and therefore, the present disclosure is not limited to the matters illustrated. In addition, when describing the present disclosure, when it is determined that a detailed description of a related known technology may unnecessarily obscure the gist of the present disclosure, the detailed description will be omitted. When the terms "include", "have", "consist of", and the like are used in this specification, other parts may be added unless "only" is used. When a component is expressed in the singular, it includes a case where the plural is included unless there is a specifically explicit description.

When interpreting components, it is interpreted as including the error range even when there is no separate explicit description.

The individual features of the various embodiments of the present disclosure may be partially or wholly combined or combined with each other, and as can be fully understood by those skilled in the art, various technical connections and operations are possible, and each embodiment may be implemented independently of each other or may be implemented together in a related relationship.

For clarity in the interpretation of this specification, the terms used in this specification are defined below.

The term "subject" as used herein may mean any subject that seeks to be provided with a user interface for a cardiac ultrasound imaging guideline from a cardiac ultrasound image. Meanwhile, the subject disclosed in this specification may be any mammal other than a human, but is not limited thereto.

As used herein, the term "cardiac ultrasound image" refers to a cardiac ultrasound image of the subject, which may be a still cut image of a single frame or a moving image composed of multiple frames.

In this case, the cardiac ultrasound image may be a 2D or 3D image.

According to a feature of the present disclosure, the cardiac ultrasound image may be a B-mode cardiac ultrasound image that is the basis for determining the measurement value. However, it is not limited thereto, and the cardiac ultrasound image may include a Doppler cardiac ultrasound image, a color Doppler cardiac ultrasound image, or an M-mode cardiac ultrasound image that enables determination of the measurement value.

As used herein, the term "anatomical structure" may refer to an anatomical structure of the heart that can be identified in a cardiac ultrasound image.

According to a feature of the present disclosure, the anatomical structure may include an area selected from the following: a right ventricle anterior wall, a right ventricle (RV), an anterior wall of aorta, an aorta, a posterior wall of aorta, a left atrium (LA), and a posterior wall of a left atrium (LA). However, the present disclosure is not limited thereto.

Here, a "parameter" or "measurement value" may mean a value, such as a measurable thickness, diameter, or the like, of a cardiac structure, and may be used interchangeably herein.

According to a feature of the present disclosure, the parameter may be at least one of an interventricular septum thickness (IVS), a left ventricular diameter (LVID), a left ventricular posterior wall diastole (LVPWD), a right ventricular outflow tract (RVOT), an aortic diameter (Ao), and a left atrium diameter (LA). However, the present disclosure is not limited thereto.

In this case, a "measurable parameter" may be defined as a measurement parameter for which a determination of a value corresponding to the parameter is possible.

According to various embodiments of the present disclosure, a prediction model trained to segment anatomical structures for frames in a cardiac ultrasound image is presented.

As used herein, the term "prediction model" may be a model configured to take as input the cardiac ultrasound image and segment the anatomical structure and output the segmented anatomical structure.

Meanwhile, the output value of the prediction model is not limited to those described above.

For example, the prediction model may be a model trained to take as input the cardiac ultrasound image and output the location (coordinates) of each anatomical structure.

As another example, the prediction model may be a model trained to take as input the cardiac ultrasound image, segment the area, and output a probability for each anatomical structure corresponding to each area.

According to a feature of the present disclosure, the prediction model may be a model further trained to determine and output a location-based connectivity meter, an uncertainty score, and further a capacity meter corresponding to a volume for each of the segmented anatomical structures.

Here, the uncertainty score may include entropy, mutual information between predictions and model posterior (for example, Bayesian Active Learning by Disagreement (BALD)) which indicates how much the probability variables of the "prediction" and "model posterior probability" are related to each other, variation ratios, and mean standard deviation, .

Additionally, the prediction model may include a model trained to input the cardiac ultrasound image, classify the type of cross-section, and output results and probabilities.

According to another feature of the present disclosure, scores for each of the connectivity meter, the uncertainty, and the capacity meter may be calculated, and quality evaluation of an image may be performed based on a combination of these scores.

In various embodiments of the present disclosure, measurable parameters may be obtained from the cardiac ultrasound image based on the positional relationship of the anatomical structures within the cardiac ultrasound image.

According to another feature of the present disclosure, a measurable image mode may be obtained from the corresponding image based on the positional relationship of the anatomical structures.

According to another feature of the present disclosure, a guideline for obtaining a measurable image mode from a corresponding image may be determined based on the positional relationship of each anatomical structure. For example, a line for obtaining a Doppler mode or color Doppler mode image, or a box for obtaining an M-mode image may be determined and displayed within a basic image. Accordingly, a user may more easily obtain images of other modes for the cardiac ultrasound image guideline. That is, images of various modes may be obtained and the measurement values may be determined using a handheld type device with a smaller screen than a general ultrasound diagnostic device.

In this case, determination of measurable parameters or determination of measurable modes may be performed by a prediction model.

That is, the prediction model may be a model further trained to segment anatomical structures in the input cardiac ultrasound image and, at the same time, predict measurable parameters or measurable modes from the image based on the segmentation results.

However, this is not limited to the above, and prediction of measurable parameters or measurable modes from images may be possible by a measurement value prediction model that exists independently of the prediction model and is trained to predict measurable parameters or modes by taking as input the segmentation results of the prediction model.

For example, the measurement value prediction model may probabilistically predict the measurable parameter or measurable mode.

According to a feature of the present disclosure, when a selection for the measurable ultrasound mode is input from a user, an image corresponding to the selected mode and values for measurable parameters within the image may be output and provided to the user.

Furthermore, the prediction model includes an output layer consisting of a plurality of nodes, where the number of nodes may correspond to the number of areas (number of classes) of predetermined anatomical structures.

According to another feature of the present disclosure, the prediction model is based on deep learning-based learning, and based on the results thereof, measurable parameters, measurable other modes (M-mode, doppler, color doppler), or the like may be determined based on a rule base, but the rule base stage is not limited thereto and may be based on machine learning-based learning.

In more various embodiments of the present disclosure, the prediction model may be a model trained to determine the probe guidance according to movement of the ultrasound probe by taking as input the cardiac ultrasound image.

In more various embodiments of the present disclosure, the prediction model may be a model trained to determine probe guidance according to ultrasound probe movement by taking as input the cardiac ultrasound image and the cross-sectional view of the image.

In this case, the prediction model may be a model trained to determine the probe guidance by taking as input the cardiac ultrasound image and the cross-sectional view of the cardiac ultrasound image.

In various embodiments of the present disclosure, the prediction model may be a model trained to probabilistically predict probe guidance.

Here, the probe guidance (probe manipulation guide) in ultrasound examination is a system that guides how to adjust the probe to obtain the correct image, and may include first probe guidance for a beginner and second probe guidance for advanced depending on the ultrasound probe movement.

In this case, the first probe guidance may be different for each mode.

In various embodiments of the present disclosure, the first probe guidance may include at least one probe operation guidance of Hold, Probe Head Tilt Down, Probe Head Tilt Up, Probe Head Rock Right, Probe Head Rock Left, Probe Rotate Clockwise, and Probe Rotate Counter-clockwise in PLAX and A4C shooting modes.

In various embodiments of the present disclosure, the first probe guidance may include at least one probe operation guidance of Hold, Probe Head Tilt Right, Probe Head Tilt Left, Probe Head Rock Down, Probe Head Rock Up, Probe Rotate Clockwise, and Probe Rotate Counter-clockwise in PSAX, A2C, A3C, and IVC shooting modes.

In various embodiments of the present disclosure, the second probe guidance may provide a more sophisticated probe operation method and include at least one probe operation guidance of Slide Up, Slide Down, Slide Left, and Slide Right to provide precise scanning of anatomical structures.

However, the probe guidance is not limited thereto and may include guidance for more various probe operations according to a cross-sectional view.

Meanwhile, the prediction model may be, but is not limited to, a model configured to determine the probe guidance based on classification of segmentation and/or cross-sectional view of anatomical structures.

In more various embodiments of the present disclosure, the prediction model may be a model trained to classify an odd cardiac ultrasound image by taking as input the cardiac ultrasound image.

That is, the prediction model may be used to maintain the correct cross-sectional view and detect abnormalities in the captured cardiac ultrasound images.

Meanwhile, the prediction model may be based on at least one algorithm selected from among DenseNet-121, U-net, VGG net, DenseNet, Fully Convolutional Network (FCN) with encoder-decoder structure, deep neural network (DNN) such as SegNet, DeconvNet, or DeepLAB V3+, Transformer such as Lawin+, SegFormer, or Swin, SqueezeNet, Alexnet, ResNet18, MobileNet-v2, GoogLeNet, Resnet-v2, Resnet50, RetinaNet, Resnet101, Inception-v3, HRNet, ResNeXt, EfficientNet. Furthermore, the prediction model may be an ensemble model based on at least two algorithm models of the above-mentioned algorithms. However, it is not limited thereto.

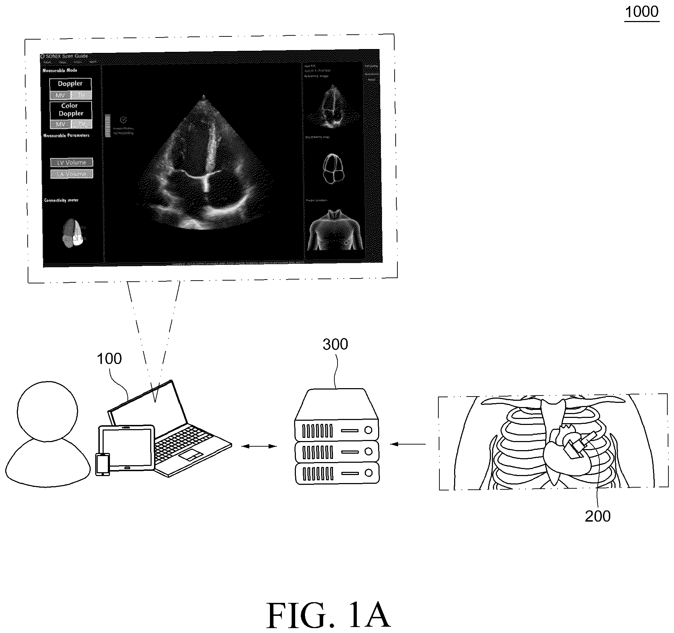

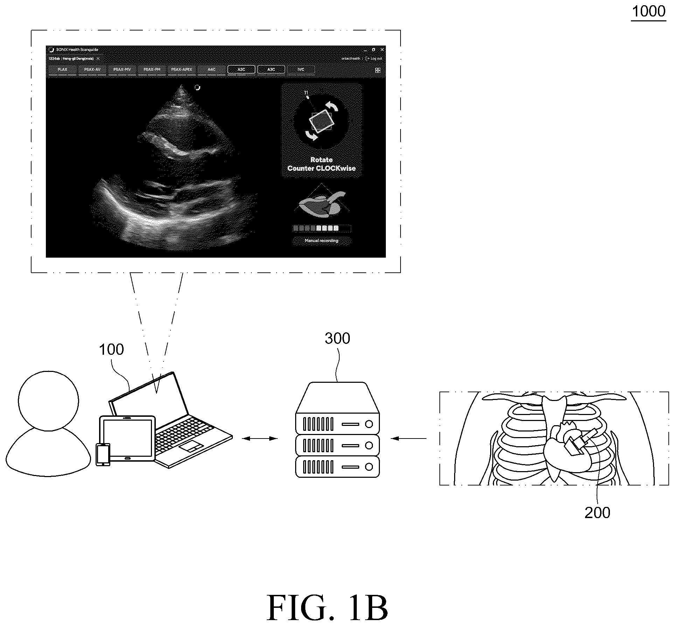

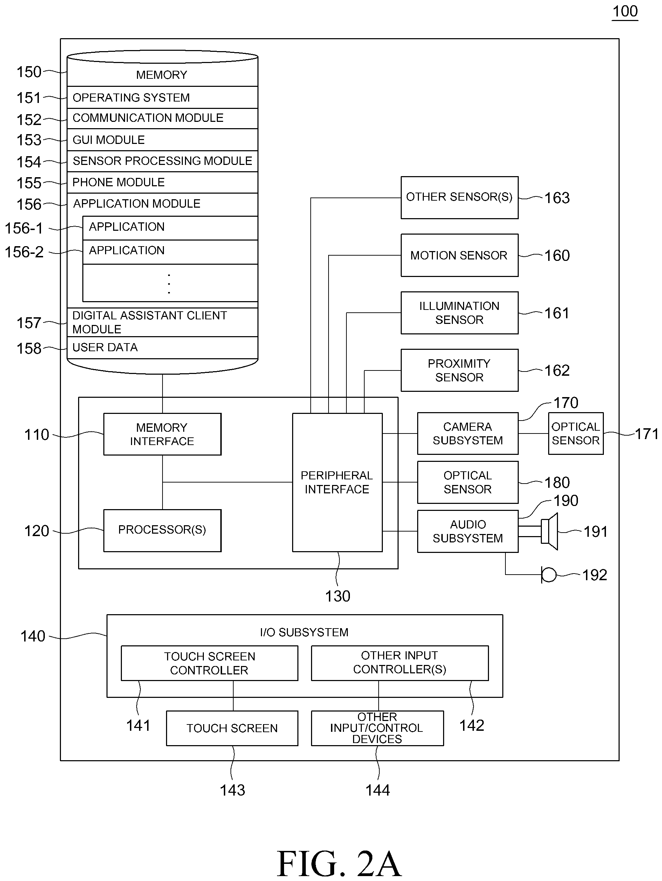

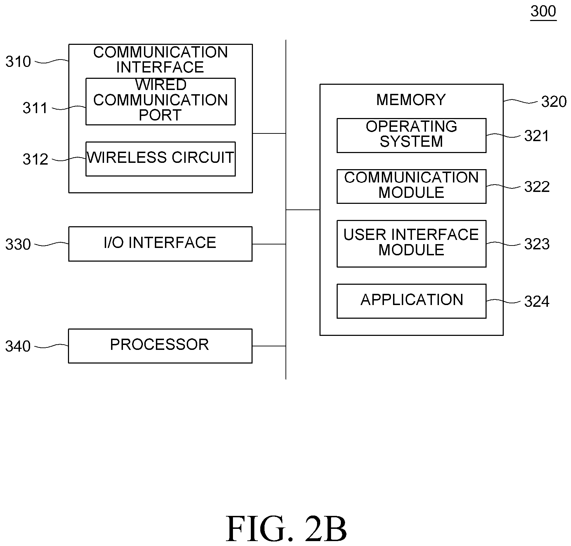

Hereinafter, with reference to FIGS. 1A and 1B, 2A and 2B, a device for providing a user interface for a cardiac ultrasound image guideline, a user device, and an interface providing system using the same according to one embodiment of the present disclosure will be described.

FIGS. 1A and 1B illustrate a system using a device for providing a user interface for a cardiac ultrasound image guideline according to various embodiments of the present disclosure. FIG. 2A illustrates an exemplary configuration of a user device that receives a user interface for a cardiac ultrasound image guideline according to one embodiment of the present disclosure. FIG. 2B illustrates an exemplary configuration of a user interface providing server for the cardiac ultrasound image guideline according to one embodiment of the present disclosure.

First, referring to FIG. 1A, a user interface providing system 1000 may be a system configured to display information associated with a measurement parameter based on a cardiac ultrasound image of a subject. In this case, the user interface providing system 1000 may be configured with a user device 100 that receives information associated with the measurement parameter, an ultrasound imaging diagnosis device 200 that provides the cardiac ultrasound image, and a user interface providing server 300 that generates the information associated with the measurement parameter based on the received cardiac ultrasound image.

In various embodiments of the present disclosure, the user interface providing server 300 may be mounted on the ultrasonic imaging diagnosis device 200, and in this case, various information associated with measurement values may be displayed on a display unit (not illustrated) of the ultrasonic imaging diagnosis device 200.

That is, a user may be provided with a user interface associated with the cardiac measurement value simultaneously with the diagnosis by using the ultrasound imaging diagnosis device 200.

In various embodiments, the user device 100 is an electronic device that provides a user interface for presenting information associated with the measurement parameter, and may include at least one of a smart phone, a tablet personal computer (PC), a laptop, and/or a PC.

The user device 100 may receive the user interface for determining the measurement parameter for a subject from the user interface providing server 300, and display the received results through a display unit (not illustrated).

The user interface providing server 300 may include a general-purpose computer, laptop, and/or data server that performs various operations for determining information associated with cardiac quantification based on the cardiac ultrasound image provided from the ultrasound imaging diagnosis device 200, such as an ultrasound diagnostic device. In this case, the user interface providing server 300 may be, but is not limited to, a device for accessing a web server providing a web page or a mobile web server providing a mobile website.

More specifically, the user interface providing server 300 receives the cardiac ultrasound image from the ultrasound imaging diagnosis device 200, performs the measurement value determination within the received cardiac ultrasound image, and provides the user interface for measurable parameters. In this case, the user interface providing server 300 may perform the prediction of the anatomical structure location within the cardiac ultrasound image using a prediction model, determine a measurable parameter therefrom, and provide a user interface related thereto.

In various embodiments, the user interface providing server 300 may determine the measurable parameter and/or measurable mode.

The user interface providing server 300 may determine the measurable parameter and/or measurable mode, the segmentation result for the anatomical structure, a guideline for image acquisition in a specific mode, a value corresponding to the measurable parameter, or the like, and provide them to the user device 100.

Referring also to FIG. 1B, in more various embodiments, the user device 100 may display the results of receiving information on guidelines for probe operation from the user interface providing server 300 through a display unit (not illustrated).

More specifically, the user interface providing server 300 may receive the cardiac ultrasound image and the cross-sectional view from the ultrasound imaging diagnosis device 200, and determine and provide the probe guidance based on the received cardiac ultrasound image. In various embodiments of the present disclosure, the user interface providing server 300 may perform a prediction on the probe guidance using the prediction model.

Furthermore, the user interface providing server 300 may determine and provide the probe guidance specific to a specific cross-sectional view.

The information provided from the user interface providing server 300 in this manner may be provided as a web page through a web browser installed on the user device 100, or may be provided in the form of an application or program. In various embodiments, such data may be provided in a form included in a platform in a client-server environment.

Next, components of the user interface providing server 300 of the present disclosure will be specifically described with reference to FIGS. 2A and 2B.

First, referring to FIG. 2A, the user device 100 may include a memory interface 110, one or more processors 120, and a peripheral interface 130. Various components within the user device 100 may be connected by one or more communication buses or signal lines.

The memory interface 110 may be connected to a memory 150 and transmit various data to the processor 120. Here, the memory 150 may include at least one type of storage medium of flash memory type, hard disk type, multimedia card micro type, card type memory (for example, SD or XD memory, or the like), RAM, SRAM, ROM, EEPROM, PROM, network storage, cloud, and blockchain data.

In various embodiments, the memory 150 may store at least one of an operating system 151, a communication module 152, a graphical user interface module (GUI) 153, a sensor processing module 154, a telephone module 155, and an application module 156. Specifically, the operating system 151 may include instructions for processing basic system services and instructions for performing hardware operations. The communication module 152 may communicate with at least one of one or more other devices, computers, and servers. The graphical user interface module (GUI) 153 may process a graphical user interface. The sensor processing module 154 may process sensor-related functions (for example, processing voice input received using one or more microphones 192). The telephone module 155 may process telephone-related functions. The application module 156 may perform various functions of the user application, such as electronic messaging, web browsing, media processing, navigation, imaging, and other processing functions. In addition, the user device 100 may store one or more software applications 156-1 and 156-2 (for example, user interface providing applications) associated with a type of service in the memory 150.

In various embodiments, the memory 150 may store a digital assistant client module 157 (hereinafter, DA client module), and thus store instructions for performing client-side functions of the digital assistant and various user data 158.

Meanwhile, the DA client module 157 may obtain the user's voice input, text input, touch input, and/or gesture input through various user interfaces (for example, I/O subsystem 140) provided in the user device 100.

Additionally, the DA client module 157 may output data in audiovisual and tactile forms. For example, the DA client module 157 may output data consisting of a combination of at least two or more of voice, sound, notification, text message, menu, graphic, video, animation, and vibration. Additionally, the DA client module 157 may communicate with a digital assistant server (not illustrated) using a communication subsystem 180.

In various embodiments, the DA client module 157 may collect additional information about the surrounding environment of the user device 100 from various sensors, subsystems, and peripheral devices to construct a context associated with the user input. For example, the DA client module 157 may provide context information along with the user input to a digital assistant server to infer the user's intent. Here, the context information that may accompany the user input may include sensor information, such as lighting, ambient noise, ambient temperature, images of the surrounding environment, video, or the like. As another example, the context information may include the physical state (for example, device orientation, device position, device temperature, power level, speed, acceleration, motion pattern, cellular signal strength, or the like) of the user device 100. As another example, context information may include information (for example, processes running on the user device 100, installed programs, past and current network activity, background services, error logs, resource usage, or the like) related to the software state of the user device 100.

In various embodiments, the memory 150 may include additional or deleted instructions, and further may include additional configurations other than those illustrated in FIG. 2A of the user device 100, or may exclude some configurations.

The processor 120 may control the overall operation of the user device 100 and execute various commands to implement an interface that provides information associated with measurement parameters by running an application or program stored in the memory 150.

The processor 120 may correspond to a computing device such as a central processing unit (CPU) or an application processor (AP). In addition, the processor 120 may be implemented in the form of an integrated chip IC such as a system on chip (SoC) in which various computing devices such as a neural processing unit (NPU) are integrated.

The peripheral interface 130 may be connected to various sensors, subsystems, and peripheral devices to provide data so that the user device 100 may perform various functions. Here, it can be understood that the user device 100 performs a certain function as being performed by the processor 120.

The peripheral interface 130 may receive data from a motion sensor 160, a light sensor (optical sensor) 161, and a proximity sensor 162, through which the user device 100 may perform orientation, light, and proximity detection functions, or the like. For another example, the peripheral interface 130 may receive data from other sensors 163 (positioning system-GPS receiver, temperature sensor, biometric sensor), through which the user device 100 may perform functions related to the other sensors 163.

In various embodiments, the user device 100 may include a camera subsystem 170 connected to a peripheral interface 130 and an optical sensor 171 connected thereto, through which the user device 100 may perform various shooting functions, such as taking pictures and recording video clips.

In various embodiments, the user device 100 may include a communication subsystem 180 coupled with a peripheral interface 130. The communication subsystem 180 may be comprised of one or more wired/wireless networks and may include various communication ports, radio frequency transceivers, and optical transceivers.

In various embodiments, the user device 100 includes an audio subsystem 190 coupled to the peripheral interface 130, the audio subsystem 190 including one or more speakers 191 and one or more microphones 192, such that the user device 100 may perform voice-activated functions, such as voice recognition, voice replication, digital recording, and telephony.

In various embodiments, the user device 100 may include an I/O subsystem 140 coupled with the peripheral interface 130. For example, the I/O subsystem 140 may control a touch screen 143 included in the user device 100 via a touch screen controller 141. As an example, the touch screen controller 141 may detect a user's contact and movement or cessation of contact and movement using any one of a plurality of touch sensing technologies, such as capacitive, resistive, infrared, surface acoustic wave technology, proximity sensor array, and the like. As another example, the I/O subsystem 140 may control other input/control devices 144 included in the user device 100 via other input controller(s) 142. As an example, the other input controller(s) 142 may control one or more buttons, rocker switches, thumb-wheels, infrared ports, USB ports, and pointer devices such as stylus.

Next, referring to FIG. 2B, the user interface providing server 300 may include a communication interface 310, a memory 320, an I/O interface 330, and a processor 340, and each component may communicate with each other through one or more communication buses or signal lines.

The communication interface 310 may be connected to the user device 100 and the ultrasound imaging diagnosis device 200 via a wired/wireless communication network to exchange data. For example, the communication interface 310 may receive the cardiac ultrasound image from the ultrasound imaging diagnosis device 200, determine measurement parameters therefrom, and transmit the user interface therefor to the user device 100.

Meanwhile, the communication interface 310 that enables transmission and reception of such data includes a communication port 311 and a wireless circuit 312, in which the wired communication port 311 may include one or more wired interfaces, for example, Ethernet, Universal Serial Bus USB, FireWire, or the like. In addition, the wireless circuit 312 may transmit and receive data with an external device via an RF signal or an optical signal. In addition, the wireless communication may use at least one of a plurality of communication standards, protocols, and technologies, for example, GSM, EDGE, CDMA, TDMA, Bluetooth, Wi-Fi, VoIP, and Wi-MAX, or any other suitable communication protocol.

The memory 320 may store various data used in the user interface providing server 300. For example, the memory 320 may store the cardiac ultrasound image, the user interface for the measurable parameters, or a prediction model trained to segment the anatomical structure within the cardiac ultrasound image.

In various embodiments, the memory 320 may include a volatile or nonvolatile storage medium capable of storing various data, commands, and information. For example, the memory 320 may include at least one type of storage medium among a flash memory type memory, a hard disk type memory, a multimedia card micro type memory, a card type memory (for example, an SD or XD memory, or the like), a RAM, an SRAM, a ROM, an EEPROM, a PROM, a network storage, a cloud, and blockchain data.

In various embodiments, the memory 320 may store a configuration of at least one of an operating system 321, a communication module 322, a user interface module 323, and one or more applications 324.

The operating system 321 (for example, an embedded operating system such as LINUX, UNIX, MAC OS, WINDOWS, VxWorks, or the like) may include various software components and drivers to control and manage general system operations (for example, memory management, storage device control, power management, or the like) and may support communication between various hardware, firmware, and software components.

The communication module 322 may support communication with other devices through the communication interface 310. The communication module 322 may include various software components for processing data received by the wired communication port 311 or wireless circuit 312 of the communication interface 310.

The user interface module 323 may receive a user's request or input from a keyboard, touch screen, microphone, or the like through the I/O interface 330 and provide the user interface on the display.

In various embodiments, the user interface module 323 may receive a selection of a measurable parameter from the user via the I/O interface 330, and provide the user interface including an area on a display in which the measurable parameter and the corresponding value are displayed.

The application 324 may include a program or module configured to be executed by one or more processors 340. Here, the application for providing information associated with the measurement parameter may be implemented on a server farm.

The I/O interface 330 may connect an input/output device (not illustrated) of the user interface providing server 300, for example, at least one of a display, a keyboard, a touch screen, and a microphone, to the user interface module 323. The I/O interface 330 may receive user input (for example, voice input, keyboard input, touch input, or the like) together with the user interface module 323 and process a command according to the received input.

The processor 340 is connected to the communication interface 310, the memory 320, and the I/O interface 330 to control the overall operation of the user interface providing server 300, and may perform various commands for providing the user interface through an application or program stored in the memory 320.

The processor 340 may correspond to a computing device such as a central processing unit (CPU) or an application processor (AP). In addition, the processor 340 may be implemented in the form of an integrated chip (IC) such as a system on chip (SoC) in which various computing devices are integrated. Alternatively, the processor 340 may include a module for calculating an artificial neural network model such as a neural processing unit (NPU).

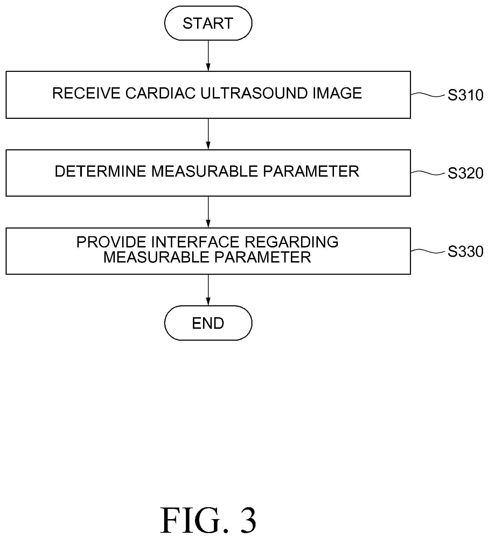

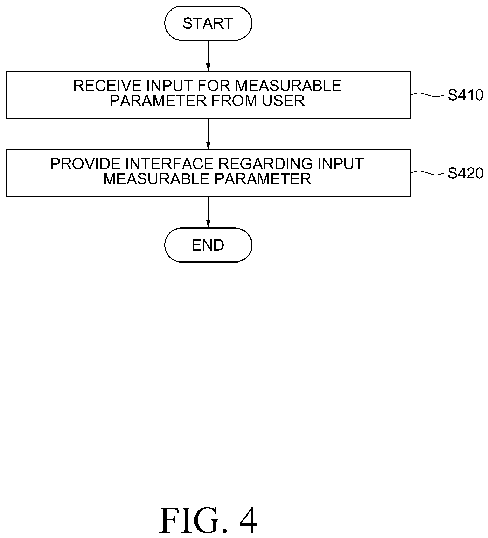

Hereinafter, a method for providing a user interface for a cardiac ultrasound image guideline according to various embodiments of the present disclosure will be specifically described with reference to FIGS. 3 and 4.

FIGS. 3 and 4 illustrate a procedure of the method for providing the user interface for the cardiac ultrasound image guideline according to one embodiment of the present disclosure.

First, referring to FIG. 3, the user interface providing procedure according to one embodiment of the present disclosure is as follows. First, the cardiac ultrasound image of a subject is received (S310). Then, the measurable parameter is determined (S320). Then, the user interface regarding the measurable parameter is provided (S330).

More specifically, in Step S310 where the cardiac ultrasound image is received, the cardiac ultrasound image of a target site, that is, a heart site, may be received.

According to a feature of the present disclosure, in Step S310 of receiving the cardiac ultrasound image, a cardiac ultrasound image of a still cut in B-mode or a cardiac ultrasound moving image including a plurality of frames may be received.

However, it is not limited thereto, and more various cardiac ultrasound images capable of determining the measurement value may be received at Step S310 of receiving cardiac ultrasound images.

Next, Step S320 is performed in which the measurable parameter is determined.

According to a feature of the present disclosure, image quality evaluation of the cardiac ultrasound image may be performed.

According to another feature of the present disclosure, the measurable parameter may be determined based on the score of each anatomical structure.

According to another feature of the present disclosure, in Step S320 where the measurable parameter is determined, an evaluation is performed for each anatomical structure, and the measurable parameter may be determined based on the evaluation.

For example, uncertainty score-based evaluation or connectivity meter-based evaluation may be performed for each determined anatomical structure, and the measurable parameter from the anatomical structure may be determined based on the results of these evaluations.

That is, even when the quality of the received ultrasound image is evaluated as low in the image quality evaluation, the evaluation of each anatomical structure may be performed, making it possible to determine the measurable parameter.

According to another feature of the present disclosure, in Step S320 in which a measurable parameter is determined, the measurable parameter may be determined from at least one mode of an M-mode cardiac ultrasound image, a Doppler cardiac ultrasound image, and a color Doppler cardiac ultrasound image based on a B-mode cardiac ultrasound image.

According to another feature of the present disclosure, in Step S320 where the measurable parameter is determined, a step of determining a measurable ultrasonic mode based on the segmentation result may be further performed.

In this case, in the step of determining a measurable ultrasound mode, the image quality evaluation is performed based on the segmentation results for each anatomical structure, and the measurable ultrasound mode may be determined based on the image quality evaluation.

In this case, the evaluation is performed on the location-based connectivity meter, uncertainty score, and further, the capacity meter corresponding to the volume for each of the segmented anatomical structures, and the scores are calculated respectively. Then, the image quality evaluation may be performed based on the total sum of each score. For example, when the sum of the scores is equal to or less than a predetermined level, it is determined as "low image quality", and when the sum is equal to or more than the predetermined level, it is determined as "good image quality" for the ultrasound mode, and the evaluation result for the image quality may be provided.

Meanwhile, in various embodiments, the connectivity meter and capacity meter may be scored based on a rule base, and in the case of entropy, residual-based score calculation may be performed. However, this is not limited thereto.

In various embodiments, guidelines for acquisition of the measurable image mode may be determined. For example, lines for acquisition of Doppler mode or color Doppler mode images, or boxes for acquisition of M-mode images, may be guided within the basic image.

Next, at Step S330 where the user interface is provided, various information determined from Step S320 where the aforementioned measurable parameter is determined is displayed and provided as the user interface.

According to a feature of the present disclosure, in Step S330 of providing the user interface, the user interface including an area in which measurable parameters and the corresponding values are displayed may be provided.

According to another feature of the present disclosure, in Step S330 of providing the user interface, a user interface including an area where the measurable parameter is displayed and an area where the ultrasonic mode is displayed may be provided.

According to another feature of the present disclosure, in Step S330 where the user interface is provided, the uncertainty score such as entropy for each anatomical structure may be displayed and provided on an area where the evaluation result is displayed.

According to another feature of the present disclosure, in Step S330 where the user interface is provided, the connectivity meter for each anatomical structure may be displayed and provided on an area where the evaluation result is displayed.

According to another feature of the present disclosure, in Step S330 of providing the user interface, a user interface including an area where a guideline is displayed may be provided.

Accordingly, the user may more easily obtain images of different modes for the cardiac ultrasound imaging guideline. That is, the images of different modes may be obtained and the measurement values may be determined using a handheld type device with a smaller screen than a general ultrasound diagnostic device.

According to another feature of the present disclosure, in Step S330 of providing the user interface, a user interface including an area displaying image quality may be provided.

Meanwhile, in various embodiments, the user interface may be called and provided based on the user input.

More specifically, referring to FIG. 4, after Step S330 in which the user interface is provided, Step S410 in which an input for the measurable parameter is received from the user is further performed.

That is, the user may input his/her selection for the parameter he/she wants to check through the provided user interface.

Next, in Step S420 where the user interface is provided, the user interface including an area where the parameter selected by the user and the value corresponding to the parameter are displayed may be provided.

According to another feature of the present disclosure, after Step S330 of providing the user interface, a step of receiving an input for the measurable ultrasound mode from the user is further performed.

That is, the user may input his/her selection for the cardiac ultrasound image mode he or she wishes to check through the provided user interface.

Accordingly, in Step S420 where the user interface is provided, the cardiac ultrasound image of a mode corresponding to the ultrasound mode selected by the user may be provided.

That is, in various embodiments of the present disclosure, various pieces of information associated with the measurement value may be called and provided to a user interface according to user input.

Accordingly, the medical staff may easily identify measurement values from acquired cardiac ultrasound images according to the user interface providing method according to various embodiments of the present disclosure, thereby quickly proceeding with the ultrasound analysis step and establishing more accurate decision-making and treatment plans.

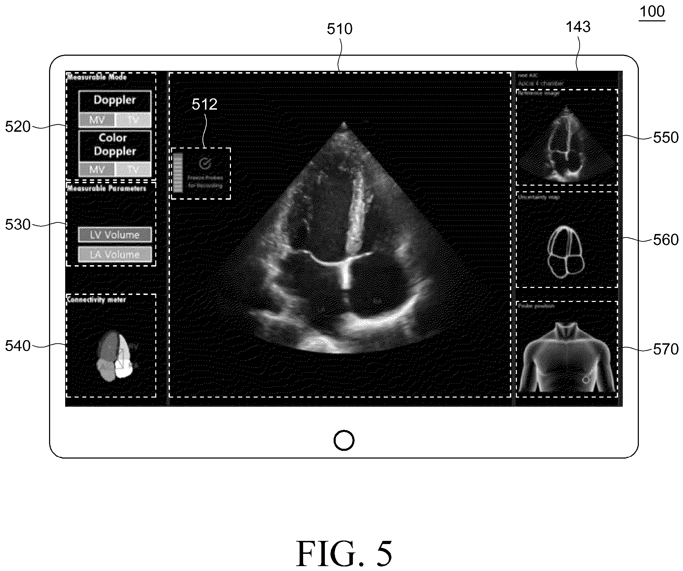

Hereinafter, the user interface for the cardiac ultrasound image guideline according to various embodiments of the present disclosure will be specifically described with reference to FIGS. 5 to 7, FIGS. 8A and 8B, FIGS. 9A to 9C, FIGS. 10 and 11.

In this case, a form in which the user device 100, the ultrasound imaging diagnosis device 200, and the user interface providing server300 each exist as independent units is described as an example, but is not limited thereto. For example, the user interface providing server 300 may be mounted on the ultrasound imaging diagnosis device 200 (for example, a handheld type diagnosis device), so that diagnosis and provision of a user interface may be implemented simultaneously in a single device.

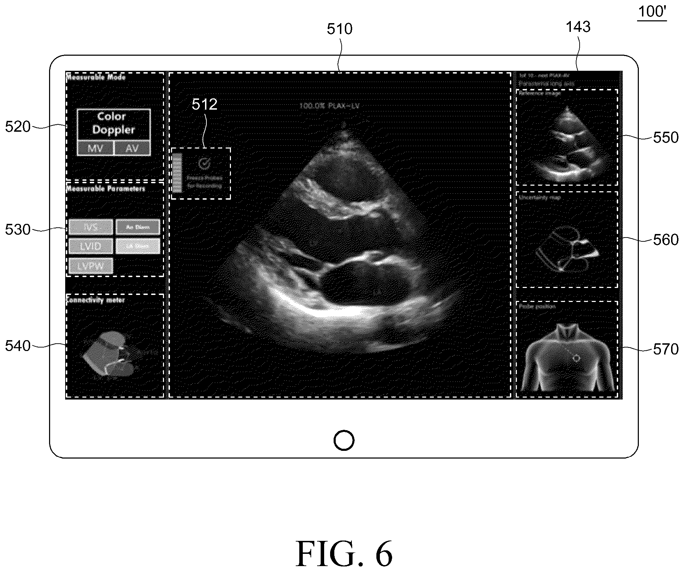

First, referring to FIGS. 5 and 6, in various embodiments, the user interface associated with the measurement value determined from the user interface providing server 300 is provided through the touch screen 143 of each of the user devices 100 and 100'.

More specifically, the user interface includes a still cut image 510 in which names of anatomical structures are displayed, which are determined to be obtainable for measurable parameters and/or modes from images acquired during diagnosis using a cardiac ultrasound probe. In this case, the still cut image 510 includes an image quality display field 512 that indicates the quality level of the image. The user may check the ultrasound mode obtainable from the still cut image 510 through the measurable mode display field 520. Furthermore, the user may check the measurement value obtainable from the still cut image 510 through the measurable parameter display field 530.

In various embodiments, the user interface includes a connectivity meter display field 540 that provides information about the evaluated connectivity meter for each of the anatomical structures of the heart within the still cut image 510.

The user may check the original image of the still cut image 510 through an original image display field 550.

In various embodiments, the user interface includes an uncertainty score display field 560 that provides information about the uncertainty score evaluated for each of the anatomical structures of the heart within the still cut image 510.

In various embodiments, the user interface includes a probe position display field 570 that tracks and provides the location of the probe.

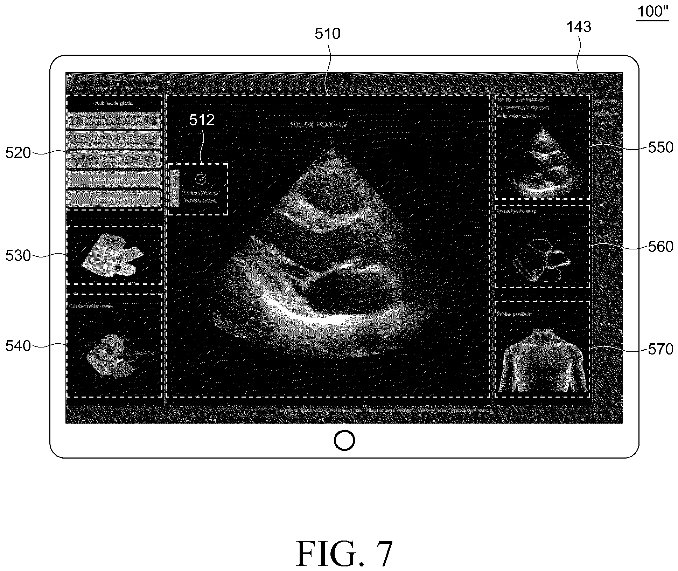

Referring further to FIG. 7, in various embodiments, the user interface may further include a volume display field 730 that displays information about segmentation results and volume for each anatomical structure of the heart within the still cut image 510.

Meanwhile, the user interface may call up and provide more various information through input received from the user.

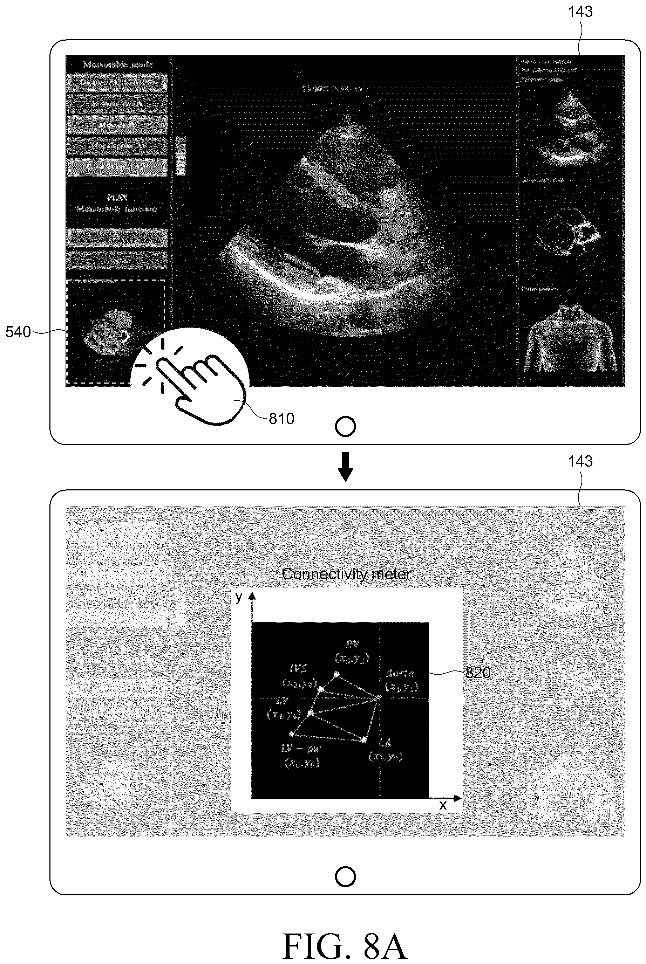

More specifically, referring to FIG. 8A, when the user inputs a selection for the connectivity meter display field 540 via the touch screen 143, a location-based connectivity meter 820 for each anatomical structure may be provided. That is, the user may check the connection relationship for each anatomical structure via the location-based connectivity meter 820.

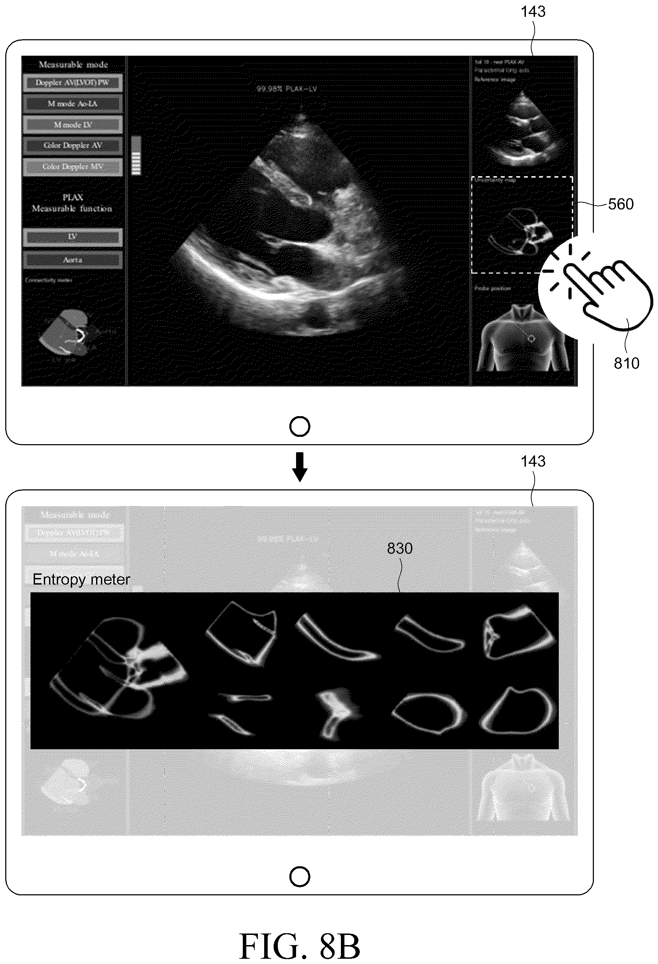

Referring further to FIG. 8B, in various embodiments, the user interface may provide the uncertainty score 830, such as an evaluated entropy meter, for each of the anatomical structures, when the user inputs a selection for the uncertainty score display field 540 via the touch screen 143. That is, the user may check the degree of uncertainty, such as the entropy meter, for each of the anatomical structures, regardless of the quality of the image, and may also obtain parameters for a specific structure.

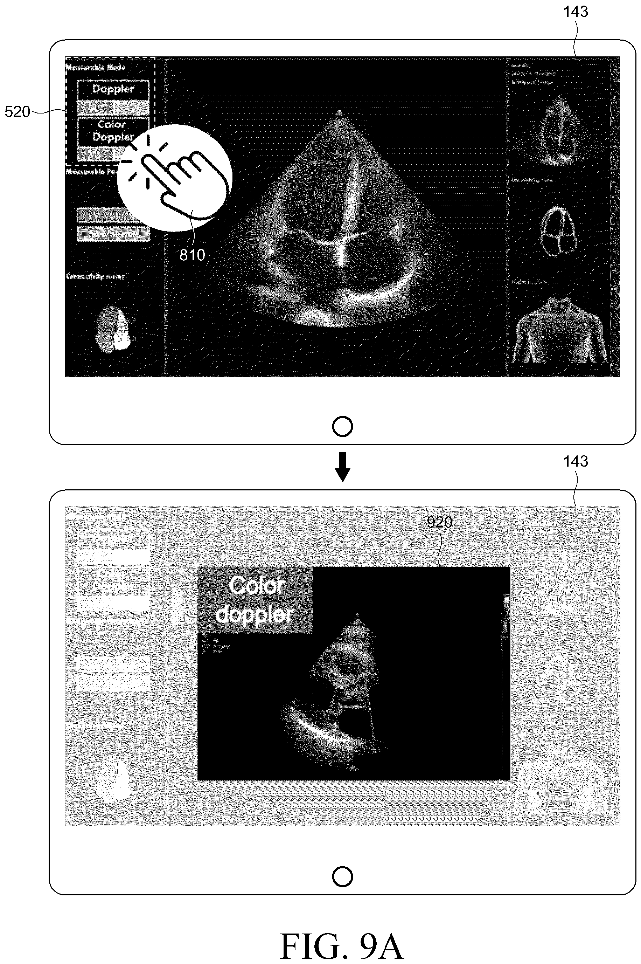

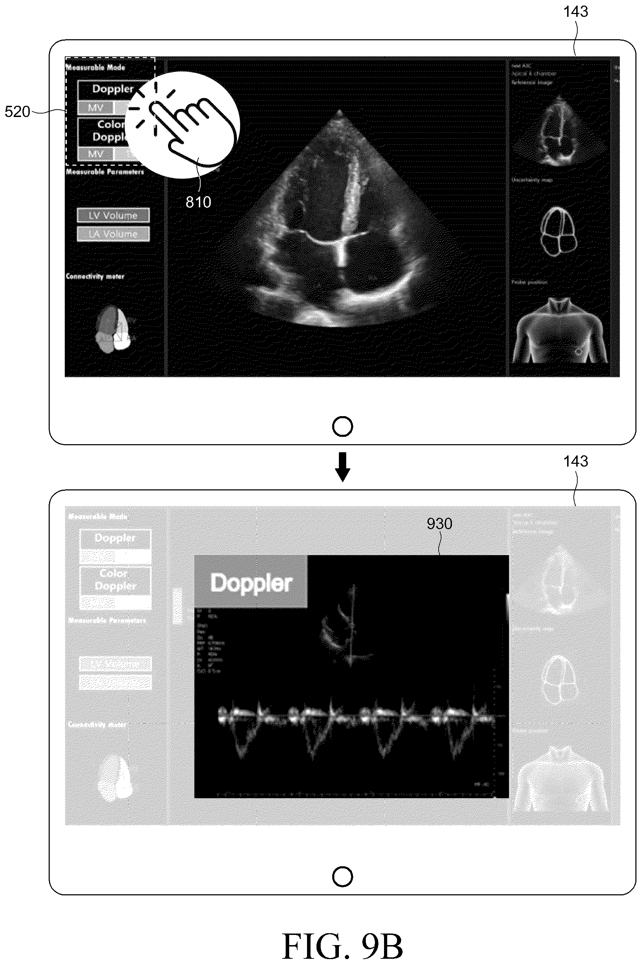

Referring further to FIGS. 9A and 9B, in various embodiments, the user interface may receive a user's selection of the measurable mode within the measurable mode display field 520 via the touch screen 143. In this case, the user interface may provide cardiac ultrasound images 920 and 930 of a mode corresponding to the selected mode. That is, the user may be able to check the cardiac ultrasound image of various modes without a procedure accompanying for a mode switch, regardless of the mode of the basic image.

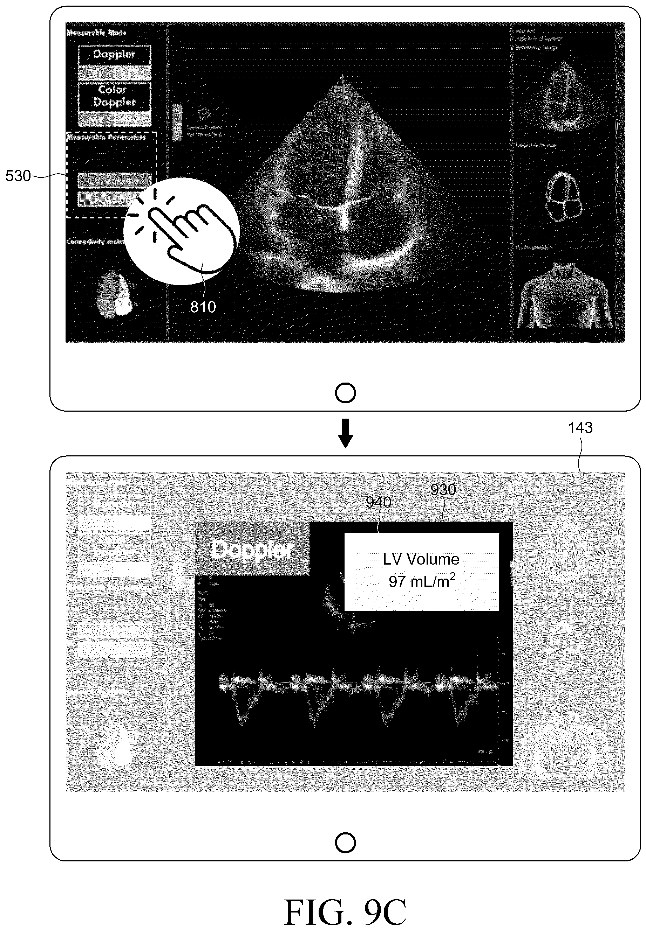

Referring further to FIG. 9C, in various embodiments, when the user inputs a selection for the measurable parameter in the measurable parameter display field 530 through the touch screen 143, the user interface may provide a value 940 corresponding to the selected parameter. In this case, the value 940 corresponding to the parameter may be provided together with the cardiac ultrasound image 930, but is not limited thereto.

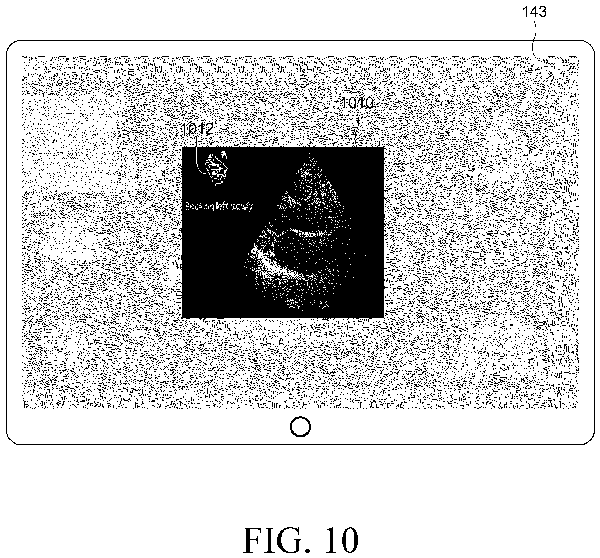

Referring further to FIG. 10, in various embodiments, the user interface may provide a guideline 1010 for image acquisition in different modes via the touch screen 143. More specifically, information regarding the movement 1012 of the probe may be provided visually to enable image acquisition in an obtainable mode.

According to a method for providing the user interface according to various embodiments of the present disclosure, the user may recognize information associated with quantification of cardiac structure more quickly.

In particular, the present disclosure is applicable to a small device including a handheld type that has limitations in performing a task for obtaining a measurement value, particularly a process for obtaining a specific image, and may provide fast and reliable information using the small device.

Meanwhile, in various embodiments of the present disclosure, the method for providing the user interface may be performed by a prediction model trained to segment a plurality of areas based on anatomical structures in the cardiac ultrasound image by taking the received cardiac ultrasound image as input.

For example, the location (coordinates) of the anatomical structures within the image may be determined by the prediction model.

In this case, the prediction model may segment at least one area selected from the following: a right ventricle anterior wall, a right ventricle (RV), an anterior wall of aorta, an aorta, a posterior wall of aorta, a left atrium (LA), and a posterior wall of a left atrium (LA) within the cardiac ultrasound image.

Furthermore, the determination of the measurable parameter, and furthermore, the determination of the measurable mode, may be performed by a prediction model configured to take as input the cardiac ultrasound image and segment and output the anatomical structure.

That is, the prediction model may be a model further trained to segment the anatomical structure of the input cardiac ultrasound image and, at the same time, predict the measurable parameter or measurable mode from the image based on the segmentation result.

However, this is not limited to the above, and prediction of measurable parameters or measurable modes from images may be possible by the measurement value prediction model that exists independently of the prediction model and is trained to predict measurable parameters or modes by taking as input the segmentation result of the prediction model.

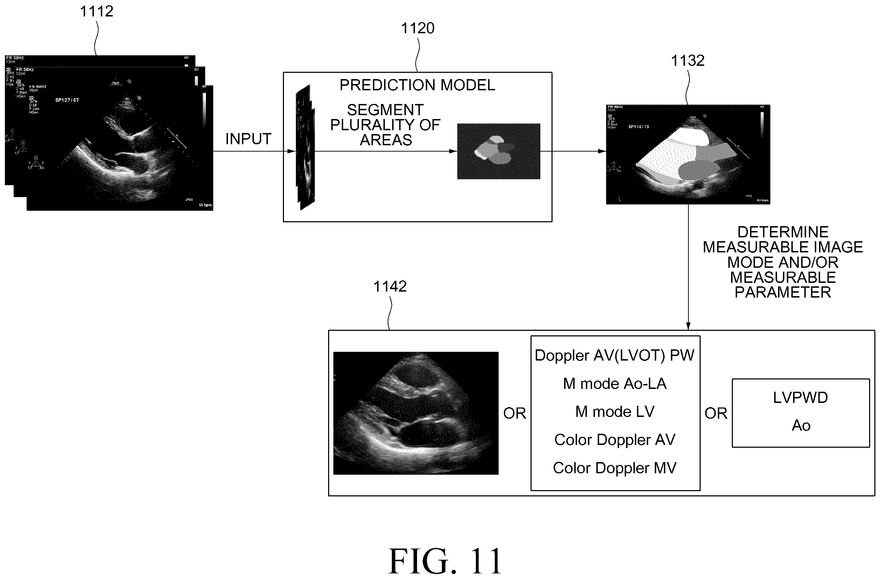

For example, referring to FIG. 11 together, when a cardiac ultrasound image 1112 is received, the cardiac ultrasound image 1112 is input into a prediction model 1120 at a step where the measurable parameter is determined. More specifically, features related to segmentation of multiple areas or anatomical structures for the input cardiac ultrasound image 1112 are extracted. After operations are performed on the extracted features, the location of the anatomical structure may be finally determined within the cardiac ultrasound image 1112. Then, measurement value-related information 1142 including measurable parameters and/or measurable modes is determined based on the location of the anatomical structure in an output segmentation result 1132. Here, the measurement value-related information 1142 may include anatomical structures within the image according to the segmentation, the mode (for example, Doppler AV (LVOT) PW, M mode Ao-LA, M mode and LV, Color Doppler AV and Color Doppler MV) measurable from the image, and the measurement parameter (for example, LVPWD and Ao) measurable from the image. However, the present disclosure is not limited thereto, and the measurement value-related information 1142 may further include values for measurable parameters, positions of the probe (not illustrated).

That is, by utilizing the prediction model, it is possible to obtain an image converted into an ultrasound image mode different from the received image, and to secure measurement values obtainable therefrom, thereby obtaining information on measurement values in more various modes.

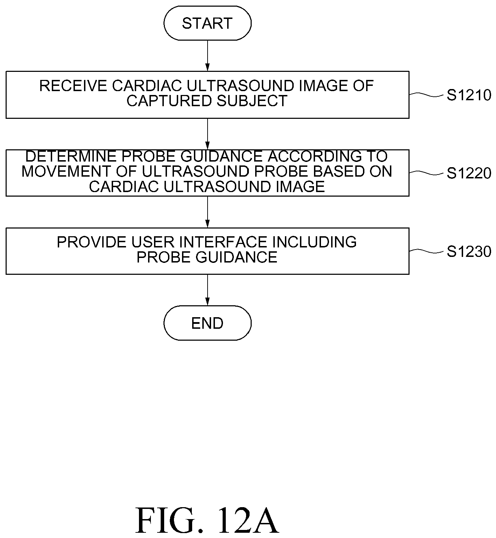

Hereinafter, with reference to FIGS. 12A to 12L, a method for providing the user interface for the guideline of the cardiac ultrasound image according to another embodiment of the present disclosure will be specifically described.

FIGS. 12A to 12L exemplarily illustrate a method for providing the user interface and the user interface according to more various embodiments of the present disclosure.

First, referring to FIG. 12A, a cardiac ultrasound image of a subject captured for providing the user interface is received (S1210), probe guidance according to movement of an ultrasound probe is determined based on the cardiac ultrasound image (S1220), and the user interface including the probe guidance is provided (S1230).

In one embodiment of the present disclosure, at the step of receiving the cardiac ultrasound image of the captured subject (S1210), the cardiac ultrasound image in the form of a video may be received. In this case, the cardiac ultrasound image may be a two-dimensional or three-dimensional ultrasound image.

In another embodiment of the present disclosure, in Step S1220 where the probe guidance is determined, the first probe guidance or the second probe guidance is determined according to movement, and in Step S1230 where the user interface is provided, an area where the first probe guidance or the second probe guidance is displayed is provided.

In this case, the first probe guidance may include at least one probe operation guidance of Hold, Probe Head Tilt Down, Probe Head Tilt Up, Probe Head Rock Right, Probe Head Rock Left, Probe Head Tilt Right, Probe Head Tilt Left, Probe Head Rock Down, Probe Head Rock Up, Probe Rotate Clockwise, and Probe Rotate Counter-clockwise.

Meanwhile, the first probe guidance may be provided in more various ways according to the ultrasound cross-sectional view.

In various embodiments of the present disclosure, the first probe guidance may include at least one probe operation guidance of Hold, Probe Head Tilt Down, Probe Head Tilt Up, Probe Head Rock Right, Probe Head Rock Left, Probe Rotate Clockwise, and Probe Rotate Counter-clockwise in PLAX and A4C shooting modes.

In various embodiments of the present disclosure, the first probe guidance may include at least one probe operation guidance of Hold, Probe Head Tilt Right, Probe Head Tilt Left, Probe Head Rock Down, Probe Head Rock Up, Probe Rotate Clockwise, and Probe Rotate Counter-clockwise in PSAX, A2C, A3C, and IVC shooting modes.

In various embodiments of the present disclosure, the second probe guidance may provide a more sophisticated probe operation method and include at least one probe operation guidance of Slide Up, Slide Down, Slide Left, and Slide Right to provide precise scanning of anatomical structures.

However, it is not limited thereto and the probe guidance may include the operations according to more various cross-sectional views.

In various embodiments of the present disclosure, the second probe guidance is defined requiring larger probe movements than the first probe guidance, and in Step S1220 of determining the probe guidance, the suitability for the first probe guidance is determined, and when the suitability for the first probe guidance is equal to or more than a predetermined level, the second probe guidance is determined. In this case, in Step S1230 of providing the user interface, the user interface including the second probe guidance is provided.

That is, the method for providing the user interface according to various embodiments of the present disclosure may provide appropriate probe guidance according to the ultrasound probe movement, and when a specific probe operation is suitable to be equal to or more than a predetermined level, more precise guidance may be additionally provided, thereby improving user experience.

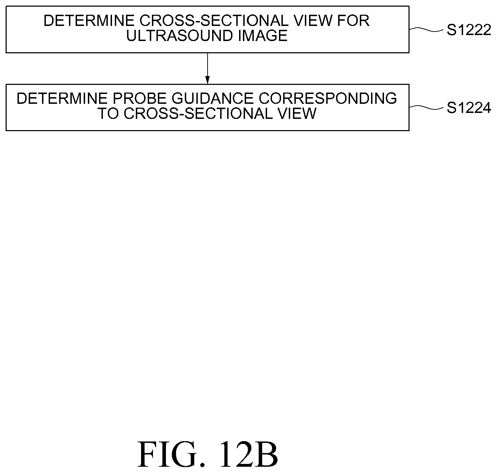

Meanwhile, referring to FIG. 12B together, in more various embodiments of the present disclosure, the cross-sectional view for the cardiac ultrasound image is determined (S1222), and the probe guidance corresponding to the cross-sectional view is determined (S1224).

Next, in Step S1230 where the user interface is provided, the user interface is provided, which includes an area where the cross-sectional view is displayed and an area where the probe guidance corresponding to the cross-sectional view is displayed.

In various embodiments of the present disclosure, the user interface may further include an area that displays the suitability according to the provision of probe guidance corresponding to the cross-sectional view.

In more various embodiments of the present disclosure, the user interface may further include an area in which the received cardiac ultrasound image is displayed.

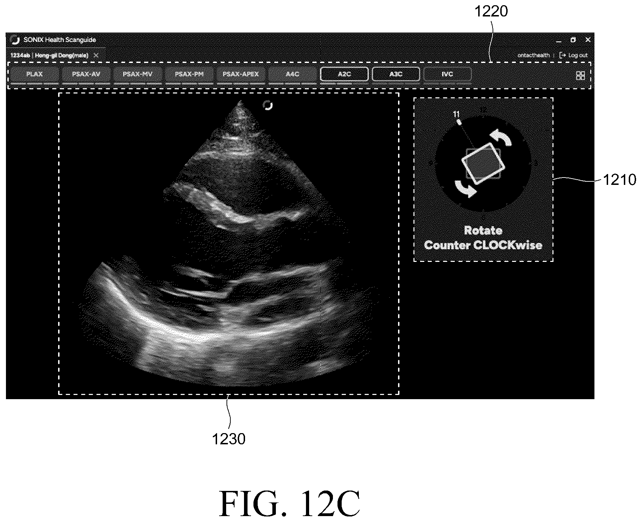

For example, referring to FIG. 12C together, in various embodiments of the present disclosure, the user interface may include an area 1210 where the probe guidance is displayed, an area 1220 where the cross-sectional view is displayed, and an area 1230 where the cardiac ultrasound image is displayed.

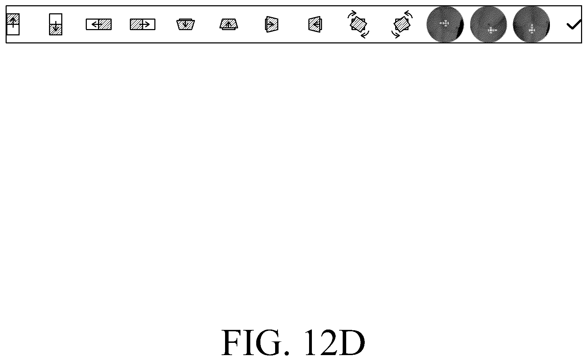

In this case, the area 1210 where the probe guidance is displayed may include the probe operation guidance suitable for the corresponding cross-sectional view. More specifically, referring to FIG. 12D together, movement for the probe operation guidance suitable for each cross-sectional view may be provided in the form of an icon, which may include the direction of movement, tilt, rotation, or the like of the probe.

Additionally, in various embodiments of the present disclosure, the area 1210 where probe guidance is displayed may provide step-by-step feedback. For example, when the user moves the probe to obtain a specific cross-sectional view, appropriate feedback according to the current probe position and operation direction may be visually provided.

In addition, the area 1210 where the probe guidance is displayed is updated in real time, and when the user correctly operates the probe to obtain an appropriate cross-sectional view, a completion mark may be provided. This allows the user to operate the probe more intuitively, and may improve the accuracy of the process for acquiring the cross-sectional view. A more specific cross-sectional view acquisition method will be described later.

In addition, an area displaying the suitability according to the provision of probe guidance corresponding to the cross-sectional view may be additionally included in the area 1220 where the cross-sectional view is displayed. In this case, the suitability may be provided in the form of a bar, but is not limited thereto, and may be provided as a numerical value such as a score or probability. Through this, the user may visually check whether the current probe operation is appropriate for the cross-sectional view, and perform more precise probe operation.

In addition, in various embodiments of the present disclosure, the user interface provides the area 1230 in which a captured cardiac ultrasound image is displayed, so that the user may obtain a more optimized cardiac ultrasound image by comprehensively considering the classification results of the cross-sectional view and probe guidance information while checking the shot cardiac ultrasound image in real time.

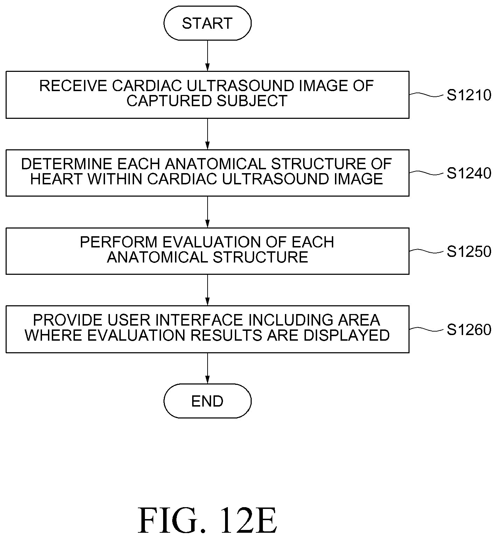

Referring to FIG. 12E, according to various embodiments of the present disclosure, the cardiac ultrasound image of the activated subject is received (S1210), each anatomical structure of the heart is determined within the received cardiac ultrasound image (S 1240), and evaluation is performed on each anatomical structure (S1250). Then, the user interface including an area where the evaluation results are displayed is provided (S1260).

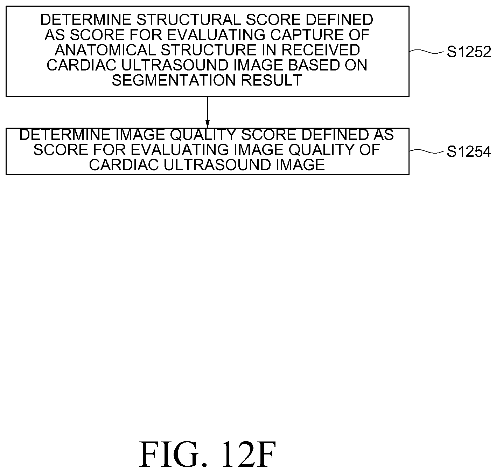

Referring to FIG. 12F together , in order to perform the evaluation of the anatomical structure in more various embodiments of the present disclosure, the structural score defined as the score for evaluating the capture of the anatomical structure in the received cardiac ultrasound image based on the segmentation result is determined (S1252), and the image quality score defined as the score for evaluating the image quality of the cardiac ultrasound image is determined (S1254).

That is, according to various embodiments of the present disclosure, each anatomical structure in the received cardiac ultrasound image is analyzed and evaluated, thereby enabling the quality of the cardiac ultrasound image to be quantitatively evaluated.

The user interface may provide the evaluation results visually so that the user may immediately recognize the suitability of the cardiac ultrasound image. In addition, by comprehensively considering the capture accuracy of the anatomical structure and the image quality, the structural score and the image quality score may be provided, thereby guiding the acquisition of more reliable ultrasound images.

In more various embodiments of the present disclosure, the structural score display area may be configured to visually display the structural score at a location corresponding to the anatomical structure based on the segmentation result, and the quality score display area may be configured to display the quality score in a bar shape.

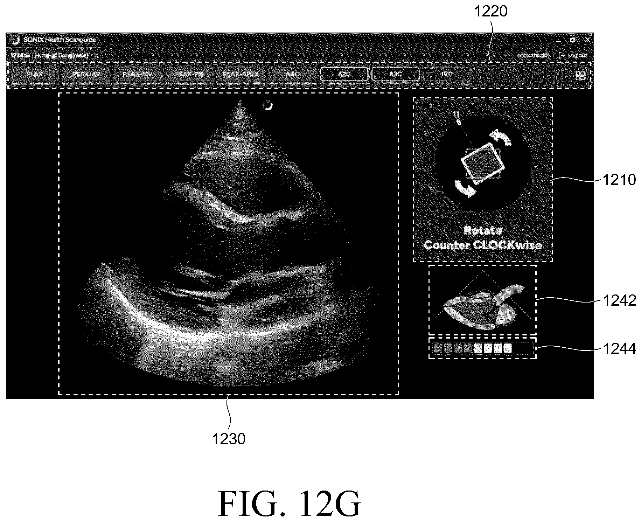

For example, referring to FIG. 12G together, a structural score display area 1242 may be configured to visually display the structural score at a location corresponding to the segmented anatomical structure within the received cardiac ultrasound image. Furthermore, a quality score display area 1244 may be configured to provide the score evaluating the quality of the cardiac ultrasound image in the form of a bar.

Through this user interface, the user may intuitively check the quality of the cardiac ultrasound image and the accuracy of capturing the anatomical structure, and operate the user interface to obtain optimal ultrasound images based on this.

However, it is not limited thereto, and the quality score may be provided in various forms depending on the characteristics of the received cardiac ultrasound image. For example, the quality score may be visually highlighted using color changes, and may be implemented in a way that provides a warning to the user when a certain threshold is exceeded. In addition, the structural score may also be expressed as a color code along with a numeric value to more clearly indicate the accuracy of capturing the anatomical structure, and may be provided with a guidance message as needed.

Returning to FIG. 12A again, after Step S1230 where the user interface is provided, when the probe is operated according to the probe guidance and an optimal cardiac ultrasound image is acquired, a step of providing a blinking effect for the area where the cardiac ultrasound image is displayed may be further performed.

Furthermore, the user interface further includes an area displaying a hold state or an area for activating an auto recording state, and in Step S1230 where the user interface is provided, the area displaying a hold state or the area for activating an auto recording state may be provided when the movement of the probe corresponds to the probe guidance.

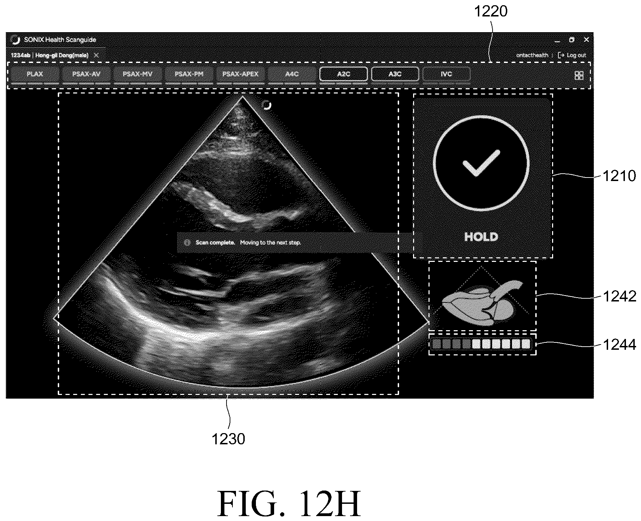

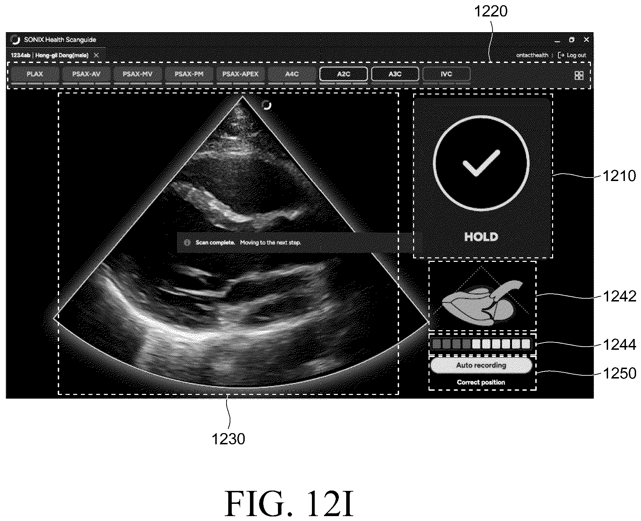

For example, referring to FIGS. 12H and 12I together, a blinking effect is applied to the area 1230 where the cardiac ultrasound image is displayed so that the user may intuitively recognize that the optimal cardiac ultrasound image has been acquired.

In addition, when the quality score display area 1244 indicating the probe operation suitability is filled with a color indicating the best score, the "HOLD" state may be activated in the area 1210 where probe guidance is displayed. At the same time, the auto recording state activation area 1250 is activated so that images may be automatically recorded in an optimal shooting state. In this way, the user interface may provide real-time visual feedback to allow the user to appropriately operate the probe and maintain an optimal shooting state.

Meanwhile, in more various embodiments of the present disclosure, the user interface further includes an area for displaying the stored cardiac ultrasound images capable of storing and managing the captured cardiac ultrasound images, in which the stored cardiac ultrasound image may include information on a captured time and cross-sectional view.

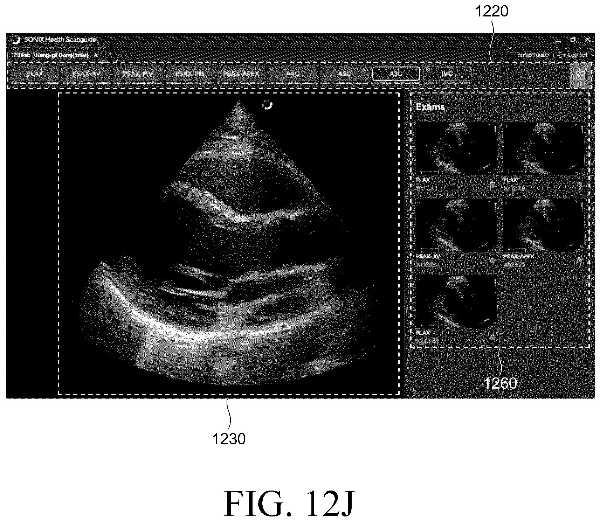

For example, referring to FIG. 12J together, it can be seen that the user interface includes an area 1260 in which stored cardiac ultrasound images are displayed, and cardiac ultrasound images captured within the area are managed in a list form.

In addition, each stored cardiac ultrasound image includes the information on the shooting time and the cross-sectional view, making it easy for the user to identify and select the recorded images. This allows the user to quickly check and compare ultrasound images shot at a specific time, and efficiently manage images for a specific cross-section view when necessary.

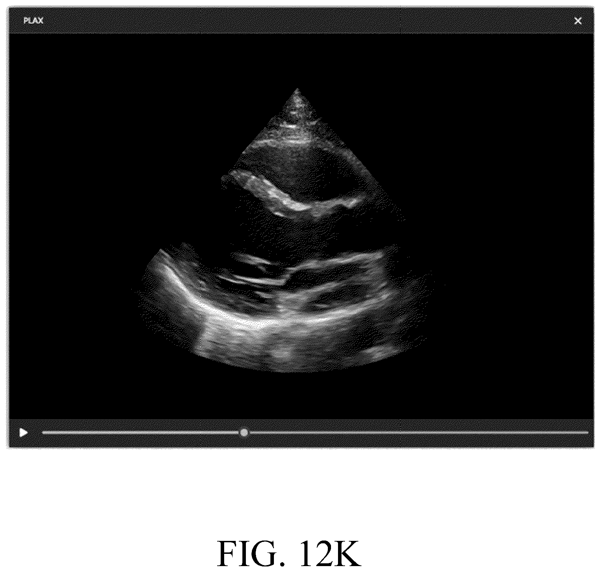

Referring further to FIG. 12K, the user interface may include the function to play back the stored cardiac ultrasound images. For example, when the specific stored cardiac ultrasound image is selected, the image may be displayed in a separate playback window, and may be configured to include a playback bar so that the user can select a desired point to play back. This allows the user to review and analyze the shot cardiac ultrasound images, and to confirm specific frames or repeatedly play them back for diagnostic purposes.

Meanwhile, the user interface is not limited thereto and may further include a settings interface for selecting options.

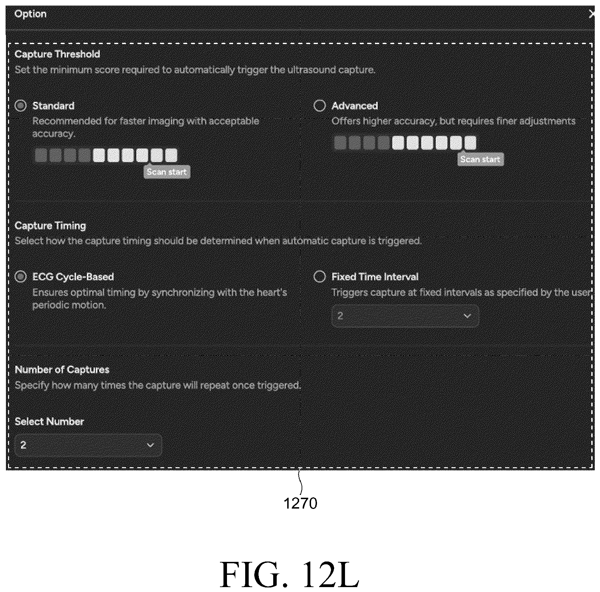

For example, referring to FIG. 12L, the user interface may include an option selection area 1270 that can set a capture threshold, capture timing, and the number of captures for acquiring the cardiac ultrasound images.

The user may adjust the capture threshold by selecting Standard or Advanced, and may choose one of ECG cycle-based synchronized to the heartbeat cycle, and fixed time interval which performs captures at regular intervals. In addition, the number of captures may be adjusted to set automatic captures to be performed as many times as desired.

This allows the user interface to be designed to support more precise ultrasound image acquisition and to allow for customized settings.

According to the method for providing the user interface according to the various embodiments described above, the present disclosure may more effectively provide cardiac ultrasound image acquisition and probe guidance.

The present disclosure may improve the accuracy of ultrasound image acquisition by providing optimal probe operation guidance according to the cross-sectional view and stepwise providing appropriate guidance according to the ultrasound probe movement. In addition, it is possible to evaluate the suitability of ultrasound images in real time by visually providing the structural score and quality score, and storage and management functions for acquired ultrasound images are provided to enable systematic image recording and analysis.

Furthermore, the present disclosure may provide intuitive feedback by activating an automatic recording function in the user interface when the probe operation is performed appropriately, or by providing the blinking effect when the cardiac ultrasound image is optimized. Through this, the user may obtain the cardiac ultrasound image more accurately and quickly, and the diagnosis and analysis process of the medical staff may be improved.

As a result, the present disclosure may provide the effect of maximizing the operational convenience for the user during the ultrasound examination process, improving the reliability of image acquisition, and enabling more efficient medical image analysis and recording.

Meanwhile, in various embodiments of the present disclosure, the method for providing the user interface may be performed by a prediction model trained to determine the probe guidance according to the movement of the ultrasound probe by taking as input the received cardiac ultrasound image.

For example, the cross-sectional view may be determined within the cardiac ultrasound image received by the prediction model, and the probe guidance suitable for that cross-sectional view may be determined.

In this case, the prediction model may be trained to provide the probe operation guidance divided into beginner guidance and advanced guidance. The beginner guidance may include operations related to the probe head movement and the probe rotation, and the advanced guidance may include operations related to rib direction movement and sliding.

Furthermore, the provision of probe guidance may be performed by the prediction model trained to classify the cross-sectional views by taking the received cardiac ultrasound image as input, and determine appropriate guidance according to the classified cross-sectional views. That is, the prediction model may be a model trained to classify the cross-sectional view of the input cardiac ultrasound image and determine the optimal probe guidance according to the cross-sectional view.

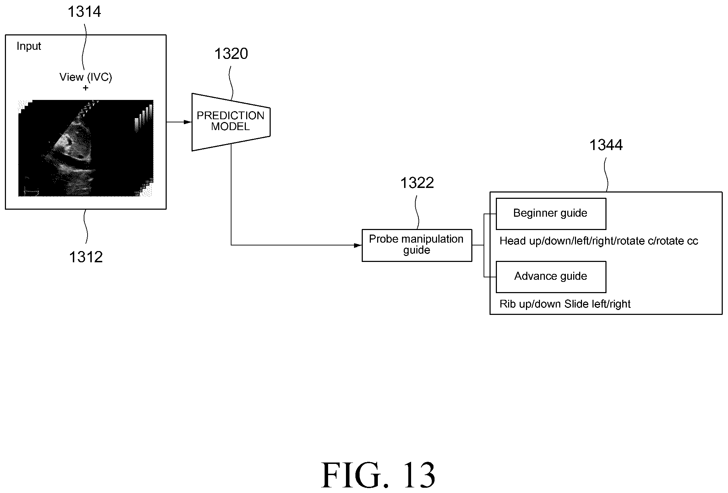

For example, referring to FIG. 13 together, when a cardiac ultrasound image 1312 is received, the cardiac ultrasound image 1312 is input into a prediction model 1320 at the step where probe operation guidance is determined. When the classification of the cross-sectional view is performed on the input cardiac ultrasound image 1312, appropriate probe guidance is determined according to the classified cross-sectional view, and then probe operation guidance information 1322 may be generated.

Next, the probe operation guidance 1344 including the beginner guidance and advanced guidance may be provided based on the output probe guidance information 1322. Here, the beginner guidance may include a basic probe operation, and the advanced guidance may include more precise probe operation.

That is, by utilizing the prediction model, appropriate probe operation guidance may be determined from the received cardiac ultrasound image, and the optimized probe operation guidance may be provided according to the ultrasound probe movement, thereby enabling more effective acquisition of cardiac ultrasound images.

Although the embodiments of the present disclosure have been described in more detail with reference to the attached drawings, the present disclosure is not necessarily limited to these embodiments, and various modifications may be made without departing from the technical idea of the present disclosure. Accordingly, the embodiments disclosed in the present disclosure are not intended to limit the technical idea of the present disclosure, but to explain it, and the scope of the technical idea of the present disclosure is not limited by these embodiments. Therefore, it should be understood that the embodiments described above are exemplary in all aspects and not restrictive. The protection scope of the present disclosure should be interpreted by the following claims, and all technical ideas within a scope equivalent thereto should be interpreted as being included in the scope of the rights of the present disclosure.

## Claims

1. A method for providing a user interface for a cardiac ultrasound image guideline implemented by a processor, the method comprising:
receiving a cardiac ultrasound image of a captured subject;
determining probe guidance according to movement of an ultrasound probe based on the cardiac ultrasound image; and
providing a user interface including the probe guidance.

2. The method according to claim 1, wherein the determining the probe guidance includes determining first probe guidance or second probe guidance according to movement, and
the user interface includes an area where the first probe guidance or the second probe guidance is displayed.

3. The method according to claim 2, wherein the first probe guidance includes at least one probe operation guidance of Hold, Probe Head Tilt Down, Probe Head Tilt Up, Probe Head Rock Right, Probe Head Rock Left, Probe Head Tilt Right, Probe Head Tilt Left, Probe Head Rock Down, Probe Head Rock Up, Probe Rotate Clockwise, and Probe Rotate Counter-clockwise, and
the second probe guidance includes at least one probe operation guidance of Slide Up, Slide Down, Slide Left, and Slide Right.

4. The method according to claim 2, wherein the second probe guidance is defined as requiring larger probe movements than the first probe guidance,
the determining the probe guidance includes
determining suitability for the first probe guidance, and
determining the second probe guidance when the suitability for the first probe guidance is equal to or more than a predetermined level, and
the providing the user interface includes providing the user interface including the second probe guidance.

5. The method according to claim 1, wherein the determining the probe guidance further includes
determining a cross-sectional view for the cardiac ultrasound image, and
determining the probe guidance corresponding to the cross-sectional view, and
the user interface includes
an area where the cross-sectional view is displayed, and
an area where the probe guidance corresponding to the cross-sectional view is displayed.

6. The method according to claim 5, wherein the user interface further includes an area for displaying suitability according to the provision of the probe guidance corresponding to the cross-sectional view.

7. The method according to claim 1, wherein the user interface further includes an area for displaying a hold state or an area for activating an auto recording state, and
the providing the user interface includes providing the area for displaying the hold state or the area for activating the auto recording state when the movement of the probe corresponds to the probe guidance.

8. The method according to claim 1, wherein the user interface further includes an area in which the received cardiac ultrasound image is displayed.

9. The method according to claim 8, further comprising, after the providing the user interface, providing a blinking effect for an area where the cardiac ultrasound image is displayed when the probe is operated according to the probe guidance to obtain an optimal cardiac ultrasound image.

10. The method according to claim 1, further comprising:
determining each of anatomical structures of a heart in the received cardiac ultrasound image; and
performing an evaluation on each of the anatomical structures,
wherein the user interface includes an area where the evaluation result is displayed.

11. The method according to claim 10, wherein the performing the evaluation includes
determining a structural score defined as a score for evaluating capture of the anatomical structure for the cardiac ultrasound image received based on a segmentation result, and
determining an image quality score defined as a score for evaluating an image quality of the cardiac ultrasound image,
wherein the area where the evaluation result is displayed includes a structural score display area and a quality score display area.

12. The method according to claim 11, wherein the structural score display area is configured to visually display the structural score at a location corresponding to the anatomical structure based on the segmentation result, and
the quality score display area is configured to display the quality score in a bar shape.

13. The method according to claim 1, wherein the user interface further includes an area for displaying a stored cardiac ultrasound image capable of storing and managing the captured cardiac ultrasound image, and
the stored cardiac ultrasound image includes information on a captured time and cross-sectional view.

14. The method according to claim 1, wherein the determining the probe guidance includes determining the ultrasound probe guidance based on the received cardiac ultrasound image by using a prediction model trained to output the ultrasound probe guidance according to the movement of the ultrasound probe by inputting the cardiac ultrasound image.

15. A device for providing a user interface for a cardiac ultrasound image guideline, the device comprising:
a communication unit configured to receive a cardiac ultrasound image of a captured subject; and
a processor and a display unit functionally connected to the communication unit,
wherein the processor is configured to determine probe guidance according to movement of an ultrasound probe based on the cardiac ultrasound image, and
the display unit is configured to display the user interface including the probe guidance.
